# EUROPEAN PATENT SPECIFICATION

(11) **EP 0 409 231 B1**
(45) Date of publication and mention of the grant of the patent: **02.05.1997**
(21) Application number: 90113856.0
(22) Date of filing: 19.07.1990
(51) Int. Cl.: G06F 19/00

(54) **Nutritive balance calculating apparatus**
Vorrichtung zum Kalkulieren des Nahrungsgleichgewichts
Dispositif pour calculer l'équilibre nutritif

(30) Priority: 19.07.1989 JP 188566/89
(43) Date of publication of application: 23.01.1991
(73) Proprietor: OTSUKA PHARMACEUTICAL CO., LTD., Chiyoda-ku Tokyo 101 (JP)
(72) Inventor: Sato, Kazuko, Naruto-shi, Tokushima 779-02 (JP); Saburomaru, Kiyoshi, Itano-gun, Tokushima 771-02 (JP); Kakumoto, Keiji, Itano-gun, Tokushima 779-01 (JP); Tani, Misako, Itano-gun, Tokushima 771-02 (JP)
(74) Representative: Hansen, Bernd, Dr. Dipl.-Chem.

(56) References cited:
- EP-A- 0 128 054
- WO-A-88/01770
- US-A- 3 841 260

## Description

### BACKGROUND OF THE INVENTION

The present invention relates to apparatus for calculating, with the use of a computer, the nutritive balance of foods taken by a person.

To continuously maintain the health of a person by his inherent natural healing power, it is required to take meals containing well-balanced nutritive elements.

However, the term of well-balanced meal is an abstract expression, and a person generally tends to select foods based on judgment made in his own way. This involves the likelihood that intake nutritive elements are not well-balanced to injure the health without knowing it.

It is therefore preferred in view of health preservation that the information of meals taken by a person is entered into a computer and that, based on accumulated data, a medical doctor or the computer passes proper judgment or gives advice according to his gender, age, body form, daily active history and the like.

Japanese Patent Unexamined Application 211760/1986 discloses a health diagnosing method comprising the steps of entering, into a computer, data of body-form measurements, physical-function measurements and medical measurements, calculating such data by the computer and supplying data of disease estimate and a prescription for optimum bodily exercise. In such a case, the advice is given while showing a display screen or a harcopy on which a list of data processing results is being displayed or printed out. For a person to which advice is given, it may be preferable that such data are given in a familiar and comprehensible manner so that he can make a deep impression on his mind.

EP-A-128054 discloses a calculator based diet management device wherein
- daily quantities of nutritive elements recommended to a user by a physician or professional are input,
- every day, the amounts of different foods taken by the user are input, whereupon the amount of nutritive elements contained in said foods is calculated and accumulated,
- after each input, the daily "credit" still "available" for each nutritive element category is obtained by comparing said accumulated actual values with said recommended values and displayed to the user.

WO-A-88/01770 shows a similar device, wherein recommended amounts for each predetermined category of food are calculated internally, taking into account the morphologic data and activity level of the user; however, only the energetic value of food is calculated, rather than nutritive elements.

US-A-3 841 250 shows a non electronic diet management device wherein each food category is associated with a colour to improve the recognition by the user.

### SUMMARY OF THE INVENTION

It is an object of the present invention as claimed to provide a nutritive balance calculating apparatus capable of judging, based on the contents of meals taken by a person, a preferred contents of meals to be taken, and capable of supplying resultant data in such manner that everybody can understand and make an impression on his mind.

The above mentioned object is achieved by an apparatus according to claim 1.

According to the invention as claimed data of daily life-activity intensity and the contents of intake meals may be entered and accumulated in the personal information memory and the amounts of intake nutritive elements may be calculated based on the concentrations of nutritive elements of each food. The calculation results of intake amounts of nutritive elements per each of a plurality of color food groups may be shown, in the form of a list, together with the required intake amounts of nutritive elements. Accordingly, the data as put in order may be explained to the user in an impressive and comprehensive manner.

The above and other features of the present invention will be apparent from the following description with reference to attached drawings.

### BRIEF DESCRIPTION OF THE DRAWINGS

Figure 1 is a flow chart showing the nutritive balance calculating procedure of apparatus in accordance with the present invention;
Figure 2 is a hardware block diagram of the nutritive balance calculating apparatus; and
Figure 3 is a graph showing results of comparison of actual intak nutritive elements with reference values.

### DESCRIPTION OF THE PREFERRED EMBODIMENTS

The following description will discuss in detail the present invention with reference to attached drawings showing an embodiment thereof.

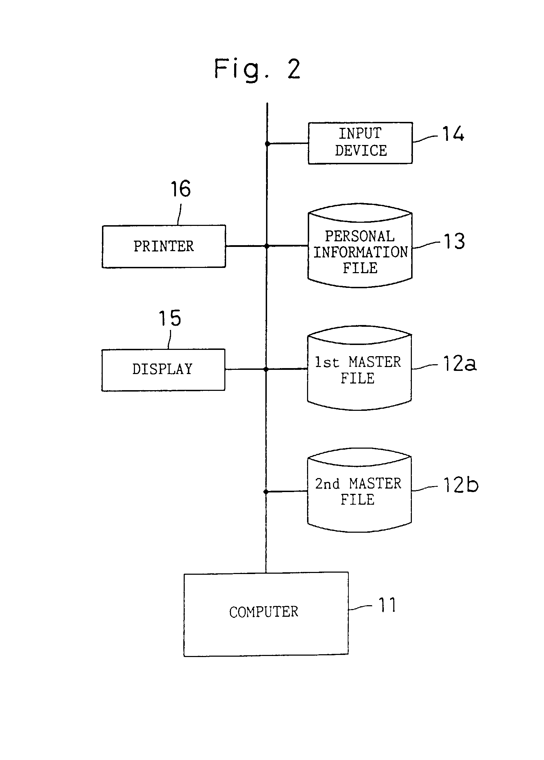

Fig. 2 is a hardware block diagram of a nutritive balance calculating apparatus of the present invention. Connected to a computer 11 through buses are a first master file 12a, a second master file 12b, a personal information file 13, an input device 14, a display 15, a printer 16 and the like.

The personal information file 13 contains personal information such as native place, personal number, family classification, name, family relationship, gender, birth date, address history and the like. The personal information file 13 may additionally contain data of an academic career and a professional career and the like, because it is statistically apparent that such data closely relate to the meal habit of a person.

A user (on whom judgment of nutritive balance is to be passed. This definition "user" will be applied to the specification in its entirety.) is adapted to write in life data and meal data, which are then entered to the apparatus through the input device 14 by an operator.

The life data contain data of body form, the degree of health, physical condition, appetite, average sleeping hours, average smoking amount, general malaise, taste for sports and the like. Such data are to be entered in answering to a questionnaire by the user. The body form refers to, for example, height and weight. As to the degree of health, physical condition and appetite, the user is to answer, in a questionnaire manner, to questions such as "Do you think that you are healty ?", "How do you think about the degree of health ?", "Are you now under a particular disease ?", "Do you have a good appetite every day ?", "Do you go to stool every day ?", "Do you have a good appetite for breakfast ?", "How about the quantity and quality of breakfast as compared with dinner ?", "Can you take action to light a lamp before making a complaint that it is dark ?", "Do you often wear a smile ?" and the like. As to the general malaise, the user is considered to have general malaise if he physically feels a bad condition. In this case, the user is to specify which part of his body is in a bad condition, and to write the symptoms in a specific manner. As to the taste for sports, the user is to answer to questions "Do you now take in a sport ?", "Which sport do you take part in ?", "How many times per month do you take in the sport ?", and "How long do you take in the sport at one time ?" Besides, if the user keeps irregular hours, he writes the cause and reasons of such irregular hours, bedtime, rising time, mealtimes and physical condition.

The meal data are to be entered every day and contain the distinction of an intake meal, meal starting time, meal taking period of time, and the names and amounts of intake foods, together with data of height, weight, bedtime of the previous night, rising time of that day, life-activity intensity of that day, impressions of that day (waking feeling, appetite, action of the bowels and moral energy). The life-activity intensity is used as a standard of the amount of activity in a daily life and divided into four stages (light, intermediate, slightly heavy and heavy). The life-activity intensity is determined based on the action in daily life and the type of profession. For example, the life-activity intensity is heavy for a coal miner. The meal distinction refers to the distinction of breakfast, lunch, dinner, snack (in the morning), snack (in the afternoon), late-night meal or the like.

The first master file 12a contains the concentrations of nutritive elements (food data) in each of foods stated on a food component calender. The nutritive elements include protein, lipid, carbohydrate, ash, mineral substances (calcium, phosphorus, iron, sodium, potassium), vitamins (A, D, B1, B2, niacin, C), salt, energy (calorie) and the like.

In addition to such data, the first master file 12a may contain the concentrations of amino acids (ILE, VAL, LEU, HIS, LYS, ARG, MET, ALA, CYS, ASP, PHE, GLU, TYR, GLY, THR, PRO, TRP, SER) of each food. The first master file 12a may also contain the required amounts of the amino acids ILE, LEU, LYS, MET, PHE, THR, TRP, and VAL per weight of 1 kg.

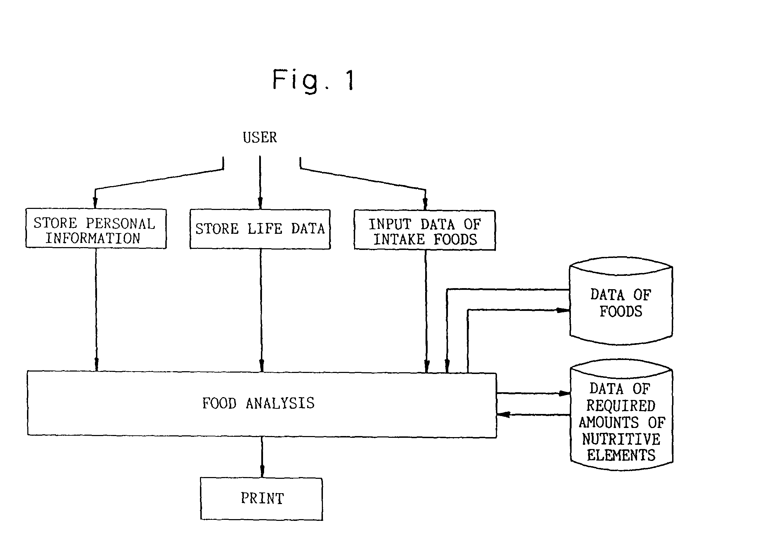

Fig. 1 is a schematic view showing a flow of processings in the nutritive balance calculating apparatus of the present invention. First of all, the user stores his personal information in the nutritive balance calculating apparatus and also stores his life data serving as standards for calculating required nutritive amounts.

Then, the user records, in a sheet, the names of foods taken every day by him. The names of intake foods are not enumerated at random, but foods are set forth as divided into three groups, i.e., red, blue and yellow groups likewise in a signal. Such classification is adopted with intent to provide the most comprehensible classification such that even an infant or a child can intuitively understand the food groups so that he can form a good meal habit, and such that an adult maintains his health to prevent a disease. The red group represents foods mainly serving as raw materials for growth and supplement of the bodily tissue, and includes fishery products, meat products, egg, milk and beans. The red-group foods are main sources of protein, niacin, vitamins B1, B2, D, calcium, phosphorus, iron, potassium and the like. The blue group represents foods for mainly maintaining the bodily function in a smooth manner, and includes green and yellow vegetables, light-colored vegetables, fruits, seaweeds, mushrooms and the like. The blue-group foods are main sources of vitamins A, C, fibers, iron, potassium, calcium, magnesium, iodine and the like. The yellow group represents foods serving as an activity energy source, and includes cereals, potatos, alcohols, oil and fats, sugar and the like. The yellow-group foods are main sources of carbohydrate and lipid.

The user is to specify the intake foods according to the classifications of the food component calender (at this time, he may specify the foods by codes), and to write the intake amounts (in gram). An example of such entry is shown in Table 1.

**Table 1**

| Meal Division | Lunch | |
|---|---|---|
| Time | Started at 12:15, Taking period of time 25 min. | |
| Menu | Rice | |
| | Sashimi of oceanic bonito | |
| | Vegetable salad | |
| Red Group | Egg | |
| | Milk | |
| | Fishery products | 80 grs. of four pieces of oceanic bonito |
| | Beans | |
| | Meat | |
| Blue Group | Green & yellow vegetables | |
| | Light-colored vegetable | 10 grs. of four pieces of Japanese radish, 20 grs. of tomato, 10 grs. of cabbage |
| | Fruit | |
| | Seaweeds & mushrooms | |
| Yellow Group | Cereals | 120 grs. of rice |
| | Potato | |
| | Sugar & sweetening agent | 10 grs of mayonnaise |
| | Oil/fat & seasening | |
| | Sweet stuff & alcohol | |

The operator of the nutritive balance calculating apparatus (which may be alternately operated by the user) enters the data supplied from the user, into the apparatus through the input device 14.

When meal data supplied from the user are accumulated for a predetermined period of time, for example a week, the intake nutritive elements are calculated based on the accumulated data. That is, intake nutritive elements per day or per meal distinction are obtained as averaging the intake amounts of nutritive elements in the respective groups. For example, there is obtained the daily average intake amount of one nutritive element, for example protein, contained in the red-group foods. Such calculation is also made for lipid, carbohydrate, mineral substance, vitamin, salt and energy. Further, such calculation is also made for blue- and yellow-group foods.

The proportions of each nutritive element held in the respective red-, blue- and yellow-groups, are also calculated. It is then understood that, for example, 70% of protein is taken from the red-group, 7% of protein from the blue-group, and 22% of proten from the yellow-group.

The foregoing results are printed out, as a list of nutritient intake amount table, in the form of a daily intake amount of each of nutritive elements per group. Examples of such a table are shown in Table 2 and Table 3.

**Table 2**

| Date July 1, 1989 | | | | | | | |
|---|---|---|---|---|---|---|---|
| Meal Distinction | Lunch | | | | | | |
| Type | Protein (g) | Lipid (g) | Carbohydrate (g) | Fiber (g) | Ca (mg) | P (mg) | Fe... (mg) |
| Red-Group | 14.3 | 18.1 | 0 | 0.0 | 3 | 112 | 2.0 .. |
| Blue-Group | 1.3 | 0.1 | 3 | 0.8 | 52 | 39 | 0.6 .. |
| Yellow-Group | 3.1 | 8.6 | 40 | 0.1 | 2 | 36 | 0.1 .. |
| Total | 18.7 | 26.8 | 43 | 0.9 | 57 | 187 | 2.7 .. |

**Table 3**

| Date July 1, 1989 | | | | | | | |
|---|---|---|---|---|---|---|---|
| For one day | | | | | | | |
| Type | Protein (g) | Lipid (g) | Carbohydrate (g) | Fiber (g) | Ca (mg) | P (mg) | Fe .. (mg). |
| Red-Group | 37.0 | 31.6 | 13 | 0.9 | 241 | 493 | 4.2 .. |
| Blue-Group | 2.7 | 0.1 | 6 | 1.4 | 129 | 72 | 1.2 .. |
| Yellow-Group | 12.5 | 12.1 | 111 | 0.5 | 36 | 131 | 1.2 .. |
| Total | 52.2 | 43.8 | 130 | 2.8 | 406 | 696 | 6.6 .. |

Tables 2 and 3 show the nutritive elements of protein, lipid, carbohydrate, fiber, Ca, phosphorus and iron. In addition to the nutritive elements above-mentioned, there are also obtained, in the same manner, the intake amounts of Na, K, vitamins A, D, B1, B2, niacin, vitamin C, salt and energy, respectively.

The intake amounts above-mentioned are then compared with the required nutritive amounts, which are determined according to the user's gender, age and height out of the life data, the life-activity intensity of the meal data, and the like. For example, there are calculated the required amounts of protein, lipid and the like which should be taken per meal by a female user of 20 year-old having a height of 150 cm of which life-activity intensity is "light". Such calculation is made with the use of, for example, numerical formula and tables. The calculated data are being stored, as the required nutritive amount data, in the second master file 12b. Examples of a table of reference values are shown in Tables 4 and 5, in which "-numeric value" refers to the upper limit, while "numeric value-" refers to the lower limit.

**Table 4**

| For one meal | | | | | | | |
|---|---|---|---|---|---|---|---|
| Type | Protein (g) | Lipid (g) | Carbohydrate (g) | Fiber (g) | Ca (mg) | P (mg) | Fe.. (mg) |
| Reference value | 22-25 | -13 | -98 | 2.5- | 180- | 180 to 200 | 4 |

**Table 5**

| For one day | | | | | | | |
|---|---|---|---|---|---|---|---|
| Type | Protein (g) | Lipid (g) | Carbohydrate (g) | Fiber (g) | Ca (mg) | P (mg) | Fe.. (mg) |
| Reference value | 65-75 | -39 | -295 | 7.5 | 560 | 560 to 600 | 12 |

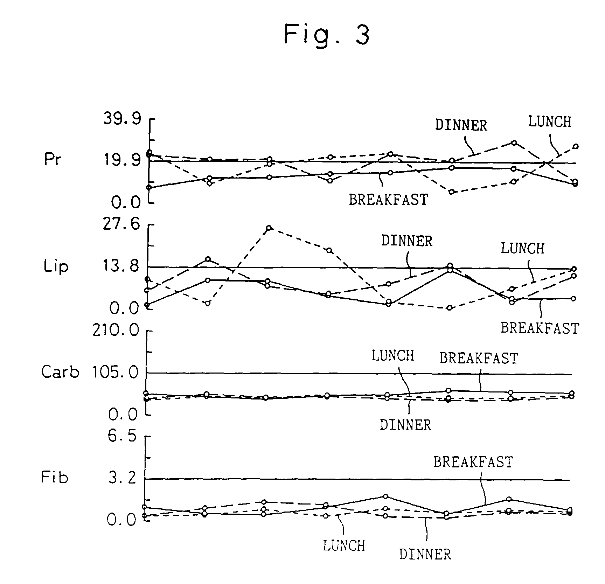

The results of comparison of the amounts of actual intake nutritive elements with the reference values are shown and printed out in the form of an excess/deficiency graph. An example of such a graph is shown in Fig. 3, which shows the actual intake amount of each of protein (Pr), lipid (Lip), carbohydrate (Carb) and fiber (Fib) per each of meal distinctios of breakfast (solid lines), lunch (dotted lines) and dinner (broken lines) for 8 days. The axis of ordinate presents the amount of intake nutrition in one meal. Each horizontal center line shows the reference value (required nutrition amount) for one meal.

This graph makes the user easily aware at a glance how the intake amounts of nutritive elements deviate from the required amounts. Provision may be made such that, if the actual intake amounts deviate from the required amounts, judgments of deficiency, considerable deficiency, excess and the like are automatically plotted.

Based on the nutrition intake tables and excess/deficiency graph above-mentioned, a medical doctor may make a diagnosis and give a numerical value presenting an evaluation rank or comments so that the user takes good recognition of proper meals. The medical doctor may also give a proper advice for future menu program.

According to the present invention, the daily average intake amount is displayed for each food group and the proportions of the daily average intake amounts held in the respective food groups are also shown. Accordingly, the data may be impressively given as put in order. Thus, the user may be readily convinced of such data with good understanding thereof. Examples of display are shown in Figs. 6 and 7.

**Table 6**

| Daily average intake amount | | | | | | | |
|---|---|---|---|---|---|---|---|
| Type | Protein (g) | Lipid (g) | Carbohydrate (g) | Fiber (g) | Ca (mg) | P (mg) | Fe... (mg) |
| Red-Group | 36.2 | 15.6 | 7 | 0.2 | 154 | 425 | 2.9.. |
| Blue-Group | 3.8 | 0.6 | 33 | 2.2 | 93 | 94 | 1.5.. |
| Yellow-Group | 11.6 | 9.2 | 118 | 0.6 | 28 | 136 | 1.1.. |
| Breakfast | 13.2 | 6.0 | 53 | 1.1 | 157 | 204 | 1.7.. |
| Lunch | 17.8 | 10.3 | 47 | 0.7 | 48 | 211 | 1.7.. |
| Dinner | 20.0 | 9.1 | 46 | 0.8 | 57 | 220 | 1.9.. |
| Snack | 0.7 | 0.2 | 12 | 0.5 | 13 | 18 | 0.1.. |
| Total | 51.6 | 25.5 | 155 | 3.0 | 273 | 652 | 5.4.. |
| Reference value | 65-75 | -39 | -295 | 7.5- | 560- | 560 to 600 | 12... |

**Table 7**

| Proportions of daily average intake amounts held in respective food groups (%) | | | | | | | |
|---|---|---|---|---|---|---|---|
| Type | Protein (g) | Lipid (g) | Carbohydrate (g) | Fiber (g) | Ca (mg) | P (mg) | Fe... (mg) |
| Red-Group | 70 | 61 | 4 | 6 | 56 | 65 | 53.. |
| Blue-Group | 7 | 2 | 21 | 71 | 34 | 14 | 27.. |
| Yellow-Group | 22 | 36 | 75 | 19 | 10 | 21 | 20... |
| Breakfast | 26 | 23 | 34 | 35 | 57 | 31 | 31... |
| Lunch | 34 | 40 | 30 | 23 | 17 | 32 | 31... |
| Dinner | 39 | 36 | 29 | 26 | 21 | 34 | 35... |
| Snack | 1 | 1 | 8 | 16 | 5 | 3 | 4... |

In addition to personal data, life data and meal data, data to be entered by the user include clinical history data, blood test data and urinary/physiology data. The clinical history data include past disease periods of time, the names of diseases, treatment progresses and the like. The blood test data include protein metabolism data such as total protein, alubumin, globulin, transferrin, prealbumin, free amino acid, uric acid, nitrogen in the form of urea, creatinine, CPK and the like; oxygen delivery-system data such as the number of red corpuscles, the number of white corpuscles, the amount of hemoglobin, hematocrit, MCV, MCH, iron, copper and the like; glycolipid metabolism data such as glucose, insulin, glucagon, triiodethyronine, total cholesterol, HDL fraction, LDL fraction, VLDL fraction, triglyceride, free aliphatic acid and the like; data of immunity and hepatic function such as the number of lymph bulbs, OKT4-8, GOT, GPT, r-GTP, ALP, bilirubin and the like; and data of vitamins and minerals such as vitamins B1 and B2, niacin, vitamins C and A, 25-OH-D, 24,25-OH (2D), Na, K, Cl, Mg, Ca, P and the like. The urinary/physiology data include urea parameters such as PH, sugar qualitative data, occult blood, protein qualitative data; and physiology parameters such as electrocardiogram, blood pressures (maximum and minimum values), lung capacity, forced erpiratory volume in one second and the like. These clinical history data, blood test data and the urinary/physiology data are effective materials based on which the user's health condition may be understood, the intake nutritions may be judged and nutrition to be taken in a future may be advised.

Having been described in detail with reference to the attached drawings, the present invention should not be limited to the embodiment above-mentioned. It should be understood that various modification may be made.

According to the nutritive balance calculating apparatus of the present invention, nutritive elements taken by the user may be calculated and the calculation results may be displayed or printed out in the form of a list presenting the intake amount of each of nutritive elements per each of a plurality of color groups. Thus, the results may be explained to the user in a familiar and comprehensible manner such that he makes a deep impression on his mind. Thus, the present invention may contritube to maintenance and promotion of personal and social health.

## Claims

1. A nutritive balance calculating apparatus for calculating, with the use of a computer (11), the nutritive balance of meals taken by a person, comprising:
a first master memory (12a) containing the concentrations of nutritive elements of each food;
a personal information memory (13) for storing the contents of intake meals;
input means (14) for entering data of daily life-activity intensity, the types of foods contained in each intake meal, and the intake amounts of said foods;
accumulating means (11) for accumulating said entered data for a predetermined period of time;
nutritive element calculating means (11) for calculating, based on said accumulated data, nutritive elements contained in said intake foods;
the apparatus being characterized by further comprising :
a second master memory (12b) containing the required amounts of nutritive elements according to personal types substantially determined by gender, age, body form and life-activity intensity;
display means (15, 16) for showing, in the form of a list, the intake amount of each of nutritive elements per each of a plurality of color groups into which said intake foods are divided according to the nutritive characteristics thereof, said intake amount being displayed together with a required nutritive amount as calculated based substantially on information of personal gender, age, body form and life-activity intensity.

2. A nutritive balance calculating apparatus according to Claim 1, wherein the color groups are composed of three color groups, i.e., a first group representing foods serving as raw materials for growth and supplement of the bodily tissue, a second group representing foods for smoothly maintaining the bodily function, and a third group representing foods serving as an activity energy source.

## Patentansprüche

1. Eine Vorrichtung für die Berechnung des Ernährungsgleichgewichts, um unter Verwendung eines Computers (11) das Ernährungsgleichgewicht der von einer Person eingenommenen Mahlzeiten zu berechnen, welche
einen ersten Stammspeicher (12a), der die Konzentrationen der Ernahrungselemente in jedem Nahrungsmittel enthält;
einen Speicher (13) für persönliche Informationen, um die Inhalte von eingenommenen Mahlzeiten zu speichern;
Eingabemittel (14), um Daten der Intensität der Lebensaktivität pro Tag, die Nahrungemitteltypen, die in jeder eingenommenen Mahlzeit enthalten sind, und die aufgenommenen Mengen der genannten Nahrungsmittel einzugeben;
Speichermittel (11), um die genannten eingegebenen Daten für eine festgelegte Zeitdauer zu akkumulieren; und
Berechnungsmittel (11) für Ernährungselemente, um auf der Basis der genannten akkumulierten Daten die Ernährungselemente zu berechnen, welche in den genannten aufgenommenen Nahrungsmitteln enthalten sind, einschließt,
wobei die Vorrichtung dadurch charakterisiert ist, daß sie darüber hinaus einen zweiten Stammspeicher (12b), welcher die Mengen der Ernährungselemente enthält, die je nach dem Typ, zu dem die Person gehört, erforderlich sind und die im wesentlichen durch das Geschlecht, das Alter, die Körperform und die Intensität der Lebensaktivität bestimmt sind; und
Displaymittel (15, 16) beinhaltet, um in Form einer Liste die aufgenommene Menge jedes Ernährungselements aus jeder einzelnen einer Vielzahl von Farbgruppen zu zeigen, in welche die genannten aufgenommenen Nahrungsmittel entsprechend ihren Nahrungsmitteleigenschaften eingeteilt sind, wobei die genannte aufgenommene Menge zusammen mit der erforderlichen Nahrungsmittelmenge gezeigt ist, welche im wesentlichen auf der Basis von Informationen betreffend das Geschlecht, das Alter, die Körperform und die Intensität der Lebensaktivität der jeweiligen Person berechnet ist.

2. Eine Vorrichtung gemäß Anspruch 1 für die Berechnung des Ernährungsgleichgewichts, wobei die Farbgruppen aus drei Farbgruppen bestehen, d.h. aus einer ersten Gruppe, die Nahrungsmittel repräsentiert, welche als Rohmaterialien für das Wachstum und die Ergänzung des Körpergewebes dienen, einer zweiten Gruppe, die Nahrungsmittel für das reibungslose Aufrechterhalten der Körperfunktionen repräsentiert, und einer dritten Gruppe, die Nahrungsmittel repräsentiert, welche als Quelle für die Aktivitätsenergie dienen.

## Revendications

1. Appareil de calcul de l'équilibre nutritif pour calculer, en utilisant un ordinateur (11), l'équilibre nutritif des repas absorbés par une personne, comprenant :
une première mémoire principale (12a) contenant les concentrations d'éléments nutritifs de chaque aliment;
une mémoire de renseignements personnels (13) pour stocker le contenu des repas absorbés;
des moyens d'entrée (14) pour entrer des données sur l'intensité de l'activité de la vie journalière, les types d'aliments contenus dans chaque repas absorbé et les quantités absorbées desdits aliments;
des moyens de stockage (11) pour stocker les données entrées pendant une période de temps déterminée au préalable;
des moyens de calcul des éléments nutritifs (11) pour calculer, d'après lesdites données stockées, les éléments nutritifs contenus dans lesdits aliments absorbés;
l'appareil étant caractérisé en ce qu'il comprend encore :
une seconde mémoire principale (12b) contenant les quantités nécessaires d'éléments nutritifs en fonction des types de personne essentiellement déterminés par le genre, l'age, la forme du corps et l'intensité de l'activité de la vie;
des moyens d'affichage (15, 16) pour représenter, sous la forme d'une liste, la quantité absorbée de chacun des éléments nutritifs pour chacun des différents groupes de couleur dans lesquels sont divisés lesdits aliments absorbés en fonction de leurs propriétés nutritives, ladite quantité absorbée étant affichée avec une quantité nutritive nécessaire calculée essentiellement d'après des renseignements concernant le genre, l'age, la forme du corps et l'intensité de l'activité de la vie de la personne.

2. Appareil de calcul de l'équilibre nutritif selon la revendication 1, dans lequel les groupes de couleur sont composés de trois groupes de couleur, à savoir un premier groupe représentant des aliments servant de matière première pour la croissance et le remplacement des tissus du corps, un second groupe représentant des aliments nécessaires pour conserver un bon fonctionnement corporel, et un troisième groupe représentant des aliments servant comme source d'énergie pour l'activité.
